Europäisches Patentamt

European Patent Office

Office européen des brevets

(19)

(11) Numéro de publication: **0 133 064**

**A1**

(12)

# DEMANDE DE BREVET EUROPEEN

(21) Numéro de dépôt: **84401320.1**

(22) Date de dépôt: **22.06.84**

(51) Int. Cl.⁴: **C 12 Q 1/00**
C 12 Q 1/52, C 12 Q 1/54
C 12 Q 1/58, C 12 Q 1/60

(30) Priorité: **23.06.83 FR 8310422**

(43) Date de publication de la demande:
**13.02.85 Bulletin 85/7**

(84) Etats contractants désignés:
**BE DE FR IT**

(71) Demandeur: **LABORATOIRES BIOTROL**
**1, rue du Foin**
**F-75140 Paris Cedex 03(FR)**

(72) Inventeur: **Cam, Gilberte**
**Rce du Parc 25 Rue du Dr. Bruel**
**F-95380 Louvres(FR)**

(72) Inventeur: **Besses, Gérard**
**2 Hameau de la Bonne**
**F-95670 Marly la Ville(FR)**

(72) Inventeur: **Uzan, Michel**
**9 Boulevard Pasteur**
**F-93320 Pavillons S/Bois(FR)**

(74) Mandataire: **Phélip, Bruno et al,**
**c/o Cabinet Harlé & Phélip 21, rue de la Rochefoucauld**
**F-75009 Paris(FR)**

(54) **Procédé pour le dosage successif de divers composants d'un prélèvement biologique à partir d'une même prise d'essai dans une même enceinte de réaction et réactifs utilisés.**

(57) L'invention concerne un procédé de dosage combiné de plusieurs éléments du sang, sur un seul échantillon et dans une seule enceinte consistant à ajouter un premier réactif pour doser par une photométrie classique un élément puis à ajouter un second réactif pour doser par une photométrie classique un second élément, les réactifs étant choisis pour qu'il n'y ait pas d'interférence entre les différentes révélations.

Application au dosage combiné du cholestérol et des triglycérides, de l'urée et du glucose et de l'alanine amino transférase et de l'aspartate aminotransférase utilisant des réactifs enzymatiques classiques mettant en jeu l'oxydation du NADH pour la détermination du premier élément.

Croydon Printing Company Ltd.

## Procédé pour le dosage successif de divers composants d'un prélèvement biologique à partir d'une même prise d'essai dans une même enceinte de réaction et réactifs utilisés.

La présente invention concerne un procédé pour doser divers constituants dans un prélèvement corporel, en n'utilisant qu'un seul échantillon du prélèvement, de volume classique, et en ajoutant successivement, dans un même récipient, des réactifs permettant de révéler quantitativement chaque constituant, lesdits réactifs étant compatibles entre eux, la révélation s'effectuant avec un appareillage classique.

De nombreux procédés de dosage des éléments du sang sont connus, mais il est habituel d'effectuer la détermination de chaque paramètre sur une nouvelle prise d'essai du sérum à étudier, et dans une nouvelle enceinte réactionnelle, afin d'éviter tous les problèmes dus à l'incompatibilité des différents réactifs, ou à l'interférence des méthodes de mesure.

Dans la demande de brevet FR n° 79 040 80, des procédés de dosage photométrique de deux des composants d'un même échantillon de sérum ont été décrits et cela dans le cas des triglycérides et du cholestérol, du glucose et de l'urée, de l'albumine et des protéines totales, de la $\gamma$-glutamyltransférase et de l'acide urique avec des réactifs déjà connus. La Demanderesse a maintenant trouvé de nouveaux réactifs, particulièrement avantageux étant donné leur stabilité et faible coût, pour effectuer de telles déterminations successivement sur un même échantillon et tels qu'il n'existe aucune incompatibilité entre leurs composants et qu'aucune interférence ne vient fausser les résultats de la

7361 EU

mesure de l'un quelconque des paramètres ; en effet, on a constaté que les résultats des mesures ne sont pas fiables quand on ajoute successivement, dans un même échantillon, certains des réactifs couramment utilisés pour les dosages séparés et qu'il est nécessaire de modifier leur composition au moins qualitative, même si les réactions chimiques mises en jeu dans les différentes étapes de révélation restent parfois les mêmes. Ainsi, selon l'invention, on effectue le dosage photométrique d'un premier constituant sur un échantillon de sang classique avec un réactif enzymatique classique, puis on ajoute dans le milieu un second réactif enzymatique classique, utile pour doser un second constituant, et simultanément au second réactif ou après la première lecture photométrique, on ajoute un système de composés pour transformer les composants présents à la fin de la première révélation et susceptibles d'interférer dans la deuxième révélation, le système et le second réactif pouvant éventuellement avoir été mélangés préalablement pour former un réactif unique ; on effectue la lecture photométrique après addition du système et du second réactif à la longueur d'onde habituelle pour le second réactif, qui peut ou non être différente de celle de la première lecture, et ajoute éventuellement un troisième réactif avec un nouveau système de composés pour transformer les composants présents à la fin de la deuxième révélation susceptibles d'interférer dans la troisième révélation et ainsi de suite. Par "transformer les composants" à la fin de la première révélation, on entend aussi bien la modification des substrats que celle des enzymes étant intervenue dans cette réaction.

Selon l'invention, les réactions révélatrices, c'est-à-dire celles qui conduisent à la formation d'un composé dont la concentration dans le milieu peut être déduite d'une mesure d'absorption de la lumière dans l'ultraviolet ou le visible, peuvent être déjà connues.

Ainsi, on peut utiliser notamment l'oxydation enzymatique du NADH en $NAD^+$, ou encore l'oxydation par le peroxyde d'hydrogène, formé de façon classique par réaction enzymatique d'un constituant biologique du sang, d'un composé ou ensemble de composés, pour donner un nouveau composé absorbant la lumière différemment.

L'invention concerne, par exemple, des procédés de dosage combiné des triglycérides et du cholestérol ou de l'urée et du glucose, couples retrouvés fréquemment dans les prescriptions médicales ; on utilise comme première réaction de révélation, pour les triglycérides ou l'urée, la transformation du NADH en $NAD^+$ en présence d'un substrat et de l'enzyme correspondante et comme seconde réaction de révélation pour le cholestérol ou le glucose, l'oxydation du mélange d'une amino-4-antipyrine, ou équivalent, avec un phénol, ou équivalent, par le peroxyde d'hydrogène formé in situ par réactions enzymatiques du cholestérol ou du glucose sanguins; pour supprimer les interférences, la révélation du second composant est effectuée, selon l'invention, en présence d'un système oxydant le NADH gênant, en $NAD^+$, non interférant, et notamment un système enzymatique qui comprend par exemple la lactate déshydrogénase associée au pyruvate ou la glutamate déshydrogénase associée à l'oxo-2-

4

0133064

glutarate et à l'ammoniac, ou la malate déshydrogénase (ou MDH) associée à l'oxaloacétate, ou encore un système oxydant non enzymatique, tel que l'ion ferrique en milieu acide dans le procédé selon l'invention, ces systèmes sont ajoutés à l'échantillon de sérum après la première lecture photométrique et avant l'addition du second réactif ou inclus dans le second réactif.

Pour le dosage combiné des triglycérides et du cholestérol, le procédé selon l'invention consiste à ajouter à un échantillon de sérum de volume habituel placé dans un récipient classique, un premier réactif A, de préférence tamponné contenant : phosphoénol pyruvate, ATP, $Mg^{++}$, NADH, glycérol kinase, pyruvate kinase, lipase et lactate déshydrogénase (ou LDH), afin de déterminer par photométrie à 340 nm la teneur en triglycérides du sérum, selon un principe connu en soi, puis à introduire dans l'échantillon ainsi traité, sans opération intermédiaire et sans changer de récipient, un second réactif B tamponné contenant: cholate, un système phénol amino-4 antipyrine ou équivalent, peroxydase, cholestérol estérase, cholestérol oxydase ainsi que pyruvate et lactate déshydrogénase, puis à effectuer une lecture de l'absorption de la solution à 500 nm pour déterminer la concentration du cholestérol dans le sérum, selon un principe connu en soi.

Selon une variante du procédé de l'invention, on utilisera un réactif B ne contenant pas de pyruvate et lactate déshydrogénase, mais on introduira ces deux constituants avant le réactif B ou simultanément à celui-ci.

Selon une autre variante de l'invention, on introduira avec le réactif ou avant celui-ci, comme système additionnel un mélange glutamate déshydrogénase et oxo-2 glutarate en milieu ammoniacal ou un mélange malate déshydrogénase et oxaloacétate ou encore des ions ferriques en milieu acide.

Les quantités utilisées des réactifs classiques sont avantageusement ceux généralement utilisés dans les dosages séparés.

La quantité de système additionnel dépend essentiellement de la quantité de premier réactif introduit ; il doit être au moins en quantité suffisante pour transformer les constituants du premier réactif qui n'ont pas réagi au cours de la révélation ; dans le cas du système enzymatique, on peut en utiliser un net excès jusqu'à 100 fois plus et, avantageusement 10 équivalents molaires du substrat choisi par rapport au NADH introduit avec le premier réactif, la quantité correspondante d'enzyme s'en déduisant de façon habituelle pour le spécialiste.

Pour le dosage combiné de l'urée et du glucose, le procédé selon l'invention implique l'utilisation d'un réactif C contenant : oxo-2 glutarate, NADH, ADP, glutamate déshydrogénase et uréase et un réactif D contenant : un système phénol amino-4 antipyrine ou équivalent, glucose oxydase, peroxydase ainsi que pyruvate et lactate déshydrogénase, ces deux derniers composés pouvant être aussi ajoutés séparément avant la seconde étape de révélation; les lectures photométriques sont effectuées successivement à 340 et 500 nm.

L'invention concerne aussi le dosage combiné des enzymes alanine aminotransférase et aspartate aminotransférase, mettant aussi en jeu l'oxydation enzymatique du NADH en NAD ; on utilise comme première étape du dosage, la révélation de l'alanine aminotransférase (ou TGP) par les réactions suivantes :

$$\text{L-analine} + \text{oxo-2-glutarate} \underset{\longrightarrow}{\overset{\text{TGP}}{\rightleftharpoons}} \text{pyruvate} + \text{L-glutamate}$$

$$\text{pyruvate} + \text{NADH} + \text{H}^+ \underset{\longrightarrow}{\overset{\text{LDH}}{\rightleftharpoons}} \text{L-lactate} + \text{NAD}$$

et comme seconde étape du dosage, la révélation de l'aspartate aminotransférase (ou TGO) par les réactions suivantes :

$$\text{L-aspartate} + \text{oxo-2-glutarate} \underset{\longrightarrow}{\overset{\text{TGO}}{\rightleftharpoons}} \text{oxaloacétate} + \text{L-glutamate}$$

$$\text{oxaloacétate} + \text{NADH} + \text{H}^+ \underset{\longrightarrow}{\overset{\text{MDH}}{\rightleftharpoons}} \text{L-malate} + \text{NAD}$$

et pour supprimer les interférences, la seconde étape est effectuée en présence d'un inhibiteur de la lactate déshydrogénase, qui la transforme en un composé inapte à catalyser la réaction du NADH sur le pyruvate, de telle sorte que seule ait lieu la réaction du NADH sur l'oxaloacétate formé par la TGO à doser.

La première lecture photométrique à la fin de la première étape est effectuée avec un appareil classique à 340 nm ; la variation de la densité optique observée au cours de la réaction est proportionnelle à la quantité de NADH transformé, donc de pyruvate formé et révèle la quantité de TGP présente dans l'échantillon - la deuxième lecture photométrique, à 340 nm indique la quantité de NADH transformé par réaction avec l'oxaloacétate, dont la quantité est proportionnelle à celle de TGO présente dans l'échantillon.

Les ensembles de réactifs utilisés dans le procédé selon l'invention sont un autre objet de l'invention.

Ces ensembles sont composés de plusieurs réac-

0133064

tifs ; un premier réactif classique, pour doser
un premier constituant du sang et un second réactif
convenablement choisi pour doser un second constituant et qui contient un système additionnel
comprenant des composés pour transformer les constituants résultants de la première révélation susceptibles d'interférer avec la seconde, ce système
additionnel peut se présenter sous la forme d'un
réactif séparé.

Les différents réactifs des ensembles pour le
dosage combiné des constituants du sang sont stables et peuvent être préparés à l'avance, ce qui
est un autre avantage de l'invention.

Le procédé et les ensembles de réactifs selon
l'invention conviennent aussi bien pour les dosages
manuels que pour les dosages automatiques. Ce
procédé combiné est d'autant plus intéressant que
les cliniciens demandent fréquemment de déterminer
à la fois le taux sanguin des triglycérides et du
cholestérol ou celui de l'urée et du glucose ou
encore celui des deux enzymes : alanine amino
transférase et aspartate amino transférase.Il est
même parfaitement concevable d'associer un nouveau
réactif à chacun de ces ensembles pour permettre
la détermination des trois composants fréquemment
demandés simultanément lors d'un même bilan sanguin
tels que, par exemple :
    - triglycérides, cholestérol et phospholipides
    - urée, glucose et urates.

Le procédé selon l'invention présente de nombreux avantages : un volume limité d'échantillon
de sang est utilisé pour chaque bilan puisqu'il
n'est pas supérieur à celui habituellement nécessaire pour doser un seul paramètre, mais aussi le nombre des manipulations est réduit et on utilise beaucoup moins de petit matériel d'analyse (cuves, pipettes....) ce qui représente évidemment une économie de coût sensible, que ce petit matériel soit à usage unique ou non.

Dans ce qui suit, des exemples de réalisation de l'invention, non limitatifs sont exposés.

Dans tout le texte, par NAD, on entend le nicotinamide adémine dinucléotide, tandis que par NADH on entend son dérivé de réduction.

EXEMPLE 1 : Dosage combiné des triglycérides et du cholestérol.

On utilise un réactif A composé des produits suivants en solution aqueuse :

| | |
|---|---|
| $Mg^{++}$ (sous forme de $MgSO_4$) | 4 mmol/l |
| NADH | 0,28 mmol/l |
| ATP (adénosine triphosphate) | 0,43 mmol/l |
| Phosphoénol pyruvate de sodium | 0,97 mmol/l |
| Glycérol kinase | $\geqslant$ 400 U/l |
| Pyruvate kinase | $\geqslant$ 600 U/l |
| Lipase | $\geqslant$ 200 000 U/l |
| Lactate déshydrogénase | $\geqslant$ 1000 U/l |
| Tampon phosphate (pH = 6,7) | 20 mmol/l |

ainsi qu'un réactif B, composé des produits suivants en solution aqueuse :

| | |
|---|---|
| Phénol | 26 mmol/l |
| Cholate de sodium | 2,4 mmol/l |
| Amino-4 antipyrine | 0,5 mmol/l |
| Peroxydase | $\geqslant$ 2000 U/l |
| Estérase | $\geqslant$ 300 U/l |
| Cholestérol oxydase | $\geqslant$ 140 U/l |
| Pyruvate de sodium | 10 mmol/l |
| Lactate déshydrogénase | $\geqslant$ 1000 U/l |
| Tampon phosphate (pH = 6,5) | 100 mmol/l |

Dans un récipient habituel, on introduit 20 µl du sérum à étudier puis 1 ml du réactif A. Après 5 minutes environ d'incubation à 37°C, on lit l'absorption de la solution à 340 nm.

On déduit la concentration du sérum en triglycérides de la différence entre l'absorption ainsi mesurée et celle d'un témoin, en se référant à un étalon.

Après cette première lecture, on introduit dans le même récipient 1 ml du réactif B; après 5 minutes environ d'incubation à 37°C on lit l'absorption à 500 nm environ. On déduit la concentration du sérum en cholestérol de la différence entre l'absorption ainsi mesurée et celle d'un échantillon témoin, sans sérum, en se référant d'un étalon.

Résultats :

On a appliqué la méthode combinée précédemment décrite à différents sérums et sur le même prélèvement snaguin, on a aussi dosé séparément, le cholestérol et les triglycérides par des méthodes classiques, c'est-à-dire sur deux échantillons différents du prélèvement.

Les résultats des dosages sont indiqués dans le tableau ci-dessous.

|  | cholestérol | |
| triglycérides | méthode classique | méthode combinée |
| --- | --- | --- |
| 2 g/l | 2,26 g/l | 2,19 g/l |
| 2 g/l | 4,02 g/l | 4,05 g/l |
| 5 g/l | 1,80 g/l | 1,85 g/l |
| 5 g/l | 4,25 g/l | 4,34 g/l |

EXEMPLE 2

Dosage combiné de l'urée et du glucose.

On utilise un réactif C, composé des produits suivants, en solution aqueuse :

| | |
|---|---|
| NADH | 0,33 mmol/1 |
| oxo-2-glutarate de sodium | 13,7 mmol/1 |
| ADP (adénosine diphosphate) | 1,2 mmol/1 |
| glutamate déshydrogénase | 250 U/1 |
| uréase | 4000 U/1 |
| tampon triéthanolamine (pH = 7,9) et un réactif D : | 75 mmol/1 |
| phénol | 26 mmol/1 |
| amino-4-antipyrine | 0,5 mmol/1 |
| glucose oxydase | 30000 U/1 |
| peroxydése | 2000 U/1 |
| pyruvate de sodium | 10 mmol/1 |
| lactate deshydrogénase | 1500 U/1 |
| tampon phosphate (pH=7,3) | 100 mmol/1 |

On introduit 10 à 20 µl du sérum à étudier dans un récipient, la quantité étant fonction de l'appareil de mesure, puis 1 ml du réactif C. On fait une mesure à 350 nm de l'évolution de l'absorption de la solution à 37°C (lecture cinétique) et on en déduit la concentration du sérum en urée par la méthode classique, puis on introduit dans le récipient 1 ml du réactif D, et après 10 minutes environ d'incubation à 37°C, on lit l'absorption de la solution à 500 nm, ce qui permet de calcuter la concentration en glucose du sérum.

On a appliqué la méthode combinée précédemment décrite à différents sérums et sur le même prélèvement sanguin mais d'autres échantillons, on a dosé séparément l'urée et le glucose par des méthodes classiques (deux échantillons différents).

Les résultats des dosages sont donnés dans le tableau ci-dessous.

| urée | glucose méthode classique | glucose méthode combinée |
|---|---|---|
| 0,39 g/l | 1,00 g/l | 0,99 g/l |
| 1,0 g/l | 2,47 g/l | 2,46 g/l |
| 0,26 g/l | 0,70 g/l | 0,68 g/l |
| 1,85 g/l | 4,31 g | 4,30 g/l |

EXEMPLE 3

Dosage combiné de l'analine aminotransférase et de l'aspartate amino transférase.

On utilise un premier réactif ayant la composition suivante, classique pour le dosage de la TGP :

(solution aqueuse)

| NADH | 0,1 mmol/l |
|---|---|
| LDH | 1100 UI/l |
| L-analine | 800 mmol/l |
| oxo-2-glutarate de sodium | 18 mmol/l |
| tampon phosphate (pH=7,4) | 80 mmol/l |

et un second réactif pour la TGO de composition :

| NADH | 0,18 mmol/l |
|---|---|
| MDH | 600 UI/l |
| L-aspartate de sodium | de 5 à 50 mmol/l |
| oxo-2-glutarate de sodium | 12 mmol/l |
| acide oxamique (sel de sodium) | de 5 à 50 mmol/l |
| tampon phosphate (pH=7,4) | 80 mmol/l |

Les résultats obtenus en appliquant ce procédé combiné en un procédé classique, dans lequel le dosage des deux éléments a été effectué sur deux échantillons du sang sont indiqués dans le tableau I. On constate que le procédé selon l'invention donne un résultat tout à fait comparable aux méthodes classiques pour le dosage de la TGO, les résultats pour la TGP étant évidemment identiques puisqu'effectués dans les conditions habituelles.

<u>ACTIVITES TGO MESUREES (en UI/l)</u>

| Echantillon N° | Procédé classique | Procédé de l'exemple 5 |
|---|---|---|
| 1 | 28 | 28 |
| 2 | 28 | 28 |
| 3 | 29,5 | 28 |
| 4 | 32,5 | 31,5 |
| 5 | 30 | 30 |
| 6 | 78 | 77 |
| 7 | 53 | 57,5 |
| 8 | 33 | 32 |
| 9 | 61 | 70 |
| 10 | 57 | 59 |
| 11 | 13 | 14 |
| 12 | 53 | 59 |
| 13 | 124 | 119 |
| 14 | 37 | 37 |
| 15 | 48 | 47 |
| 16 | 15 | 14 |
| 17 | 20 | 19 |
| 18 | 41,5 | 45 |
| 19 | 15 | 14 |
| 20 | 16 | 14 |
| 21 | 13 | 10,5 |
| 22 | 17 | 14 |
| 23 | 23 | 23 |

Une analyse statistique des résultats figurant dans le tableau I a donné un coefficient de corrélation de r = 0,993 et une droite de régression y = 0,976 x + 0,7.

## REVENDICATIONS

1. Procédé de dosage de plusieurs constituants sanguins sur un seul échantillon de sang consistant à ajouter un premier réactif pour mesurer la concentration d'un premier constituant par une méthode photométrique classique puis à ajouter un second réactif pour mesurer la concentration d'un second constituant par une méthode photométrique classique et ainsi de suite, caractérisé en ce que les réactifs sont choisis pour qu'il n'y ait pas d'interférence entre les révélations des différents constituants.

2. Procédé selon la revendication 1, caractérisé en ce qu'on utilise un premier réactif enzymatique classique et un deuxième réactif enzymatique classique auquel on ajoute un système de composés pour transformer les composants présents à la fin de la première révélation et susceptibles d'interférer dans la deuxième révélation.

3. Procédé selon les revendications 1 et 2 dans lequel on effectue une première mesure photométrique qui dépend de l'oxydation enzymatique du NADH en NAD$^+$ et une seconde, de l'oxydation d'un mélange d'amino-4 antipyrine, ou analogues, et d'un phénol, substitué ou non, par le peroxyde d'hydrogène formé in situ par réaction enzymatique, caractérisé en ce que, simultanément, ou avant ce deuxième réactif classique, est ajouté au milieu réactionnel un ensemble de composés formant un système oxydant du NADH.

4. Procédé selon l'une quelconque des revendications précédentes, caractérisé en ce que le système oxydant est un système enzymatique.

5. Procédé selon la revendication 4, caracté-

14 0133064

risé en ce que le système enzymatique est choisi parmi la lactate déshydrogénase associée au pyruvate ou la glutamate déshydrogénase associée à l'oxo-2 glutarate et l'ammoniac, ou la malate déshydrogénase associée à l'oxaloacétate.

6. Procédé selon l'une des revendications 1 à 3, caractérisé en ce que le système oxydant est non-enzymatique.

7. Procédé selon la revendication 6, caractérisé en ce que le système oxydant est le mélange $Fe^{3+}$ et $H^+$.

8. Procédé selon l'une quelconque des revendications précédentes pour le dosage combiné des triglycérides et du cholestérol, dans lequel on ajoute à l'échantillon de sérum un premier réactif à base de NADH, ATP, $Mg^{++}$, phosphoénol pyruvate, glycérol kinase, pyruvate kinase, lipase et lactate déshydrogénase et fait une lecture photométrique à 340 nm environ pour déterminer les triglycérides, caractérisé en ce que le deuxième réactif est ajouté après la lecture et qu'il est à base de cholate, amino-4 antipyrine, phénol, peroxydase, cholestérol estérase, cholestérol oxydase et en ce qu'avant ou simultanément au deuxième réactif, on ajoute un système oxydant du NADH et que l'on effectue la détermination du cholestérol par lecture photométrique à 500 nm environ.

9. Procédé selon l'une des revendications 1 à 7 pour le dosage combiné de l'urée et du glucose dans lequel on ajoute à l'échantillon à doser un premier réactif à base de NADH, ADP, oxo-2 glutatrate, glutamate déshydrogénase et uréase et détermine l'urée par photométrie à 340 nm, caractérisé en ce que l'on ajoute dans le milieu, après la lecture, un deuxième réactif qui contient de l'amino-4 antipyrine, un phénol, de la glucose oxydase, de la peroxydase et en ce qu'avant ou simultanément à celui-ci, on ajoute un système oxydant du NADH, et que l'on détermine le glucose par photométrie à 500 nm environ.

10. Procédé selon la revendication 1 ou 2, dans lequel on effectue le dosage combiné de l'alanine amino transférase et de l'aspartate amino transférase par addition d'un premier réactif qui comprend de la L-alanine, de l'oxo-2 glutarate, du NADH et de la lactate déshydrogénase et lecture photométrique à 340 nm environ puis par addition d'un second réactif qui comprend du L-aspartate, de l'oxo-2 glutarate, du NADH et de la malate déshydrogénase, suivie d'une lecture photométrique à 340 nm environ, caractérisé en ce que le second réactif comprend en outre un inhibiteur de la lactate déshydrogénase.

11. Procédé selon la revendication 10, caractérisé en ce que l'inhibiteur est choisi parmi l'acide oxamique et l'acide oxalique.

12. Procédé selon l'une quelconque des revendications précédentes, caractérisé en ce que les réactifs classiques sont tamponnés.

13. Ensemble de réactifs pour le dosage combiné par photométrie classique des constituants du sang par un procédé selon l'une quelconque des revendications 1 ou 2.

14. Ensemble de réactifs pour le dosage combiné des triglycérides et du cholestérol par un procédé selon l'une quelconque des revendications 1 à 8 et 12.

15. Ensemble de réactifs pour le dosage combiné de l'urée et du glucose par un procédé selon l'une quelconque des revendications 1 à 7, 9 et 12.

16. Ensemble de réactifs pour le dosage combiné de l'alanine amino transférase et de l'aspartate amino transférase par un procédé selon l'une quelconque des revendications 1, 2 et 10 à 12.

**RAPPORT DE RECHERCHE EUROPEENNE**

EP  84 40 1320

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| D,A | FR-A-2 449 280 (VITATRON SCIENTIFIC B.V.) * en entier * | 1-9,14 ,15 | C 12 Q    1/00<br>C 12 Q    1/52<br>C 12 Q    1/54<br>C 12 Q    1/58<br>C 12 Q    1/60 |
| | --- | | |
| A | EP-A-0 041 366 (HITACHI LTD.) * abrégé; page 6, réaction 8; revendication 1 * | 1,3 | |
| | --- | | |
| A | US-A-3 925 162 (T. KANNO) * en entier * | 1-9,14 ,15 | |
| | --- | | |
| A | GB-A-2 050 601 (AMERICAN MONITOR CORP.) * abrégé; revendication 1 * | 7 | |
| | --- | | **DOMAINES TECHNIQUES RECHERCHES (Int. Cl. ³)** |
| A | FR-A-2 433 579 (COULTER ELECTRONICS INC.) * pages 7,14,15; revendications 1,3,5 * | 1-3,10 -12,16 | C 12 Q<br>G 01 N |
| | --- | | |
| P,A | FR-A-2 532 326 (BIOTECHNIKA) * en entier * | 1-9,14 ,15 | |
| | --- | | |
| A | EP-A-0 008 341 (BOEHRINGER MANNHEIM GmbH) * abrégé; revendications * | 2-4,10 -12,16 | |
| | ----- | | |

Le présent rapport de recherche a été établi pour toutes les revendications

| Lieu de la recherche | Date d'achèvement de la recherche | Examinateur |
|---|---|---|
| LA HAYE | 28-09-1984 | OSBORNE H.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82